# EUROPEAN PATENT SPECIFICATION

(11) **EP 3 092 898 B1**
(45) Date of publication and mention of the grant of the patent: **04.07.2018**
(21) Application number: 15461533.0
(22) Date of filing: 13.05.2015
(51) Int. Cl.: A01P 13/00, A01N 25/30, A01N 39/04, C07C 229/12, C07C 229/22, C07C 233/36, C07C 59/70

(54) **HERBICIDAL IONIC LIQUIDS WITH BETAINE TYPE CATION**
HERBIZID WIRKENDE IONISCHE FLÜSSIGKEITEN DIE BETAINE-TYP KATIONEN ENTHALTEN
LIQUIDES IONIQUES HERBICIDES COMPRENANT UN CATION DE TYPE BÉTAINE

(43) Date of publication of application: 16.11.2016
(73) Proprietor: Przedsiebiorstwo Produkcyjno-Consultingowe ADOB sp. z o.o. sp. k., 61-070 Poznan (PL)
(72) Inventor: NAWROCKI, Adam, 61-306 Poznan (PL); OLSZEWSKI, Radoslaw, 61-038 Poznan (PL); PERNAK, Juliusz, 60-573 Poznan (PL); PRACZYK, Tadeusz, 62-030 Lubon (PL); NIEMCZAK, Michal, 88-100 Inowroclaw (PL); CZERNIAK, Kamil, 62-610 Sompolno (PL)
(74) Representative: Sitkowska, Jadwiga

(56) References cited:
- WO-A1-2009/004044
- WO-A1-2011/034444
- ANUBHUTI SETH, ANITA GARG AND M.L. SHARMA: "Studies on the synthesis of ammonium salts of 2,4-dichlorophenoxyacetic acid (2,4-D) to enhance its bioregulating potential", J. INDIAN CHEM. SOC., vol. 88, March 2011 (2011-03), pages 405-414, XP009185404,

## Description

### Field of the invention

The present invention relates to novel compounds, quaternary ammonium salts containing anion of herbicidally active compound 2,4-D or MCPA and betaine type cation, and herbicidal compositions comprising novel compounds. The compounds of the invention are ionic liquids and find the application as herbicides for plant protection.

### Prior art

Weeds due to their negative interactions with cultivated plants contribute largely to reduction of amount and quality of crops. Protection of cultivated plants from competitive interaction of weeds not only is necessary to secure crops but also contributes to limitation of dispersal of unwanted vegetation in subsequent growing seasons.

There are a wide range of commercially available active substances with different mechanisms of action for effective weed control, from popular phenoxyacids to bioherbicides based on microorganisms. Apart from obvious benefits, herbicides have also certain disadvantages, such as for example long persistence in soil of some active substances and their metabolites, leaching of herbicides into groundwater, negative interaction on non-target organisms, and selection of weeds biotypes resistant to active substances used.

One of the group of herbicides most commonly manufactured and used are phenoxycarboxylic acids, such as (4-chloro-2-methylphenoxy)acetic acid known as MCPA and (2,4-dichlorophenoxy)acetic acid known as 2,4-D. They have been widely used for protection of winter-sown and spring-sown cereals, potatoes, linum, fruit trees and for conservation of grasslands. They can be used in different formulations as sodium, potassium, dimethylammonium, or isopropylammonium salts, or esters (C. D. S. Tomlin, The Pesticide Manual, a World Compendium, BCPC: Fifteenth Edition, 2009). To enhance herbicidal effect, MCPA and 2,4-D can be used in mixtures with other active substances, such as for example dichloroprop [2-(2,4-dichlorophenoxy)propanoic acid], mecoprop [2-(4-chloro-2-methylphenoxy)propanoic acid], dicamba [(3,6-dichloro-2-methoxybenzoic acid)] and fluroxypyr [(4-amino-3,5-dichloro-6-fluoro-2-pyridinyl)oxy]-acetic acid, or phosphonic acid based herbicides, such as glyphosate.

A. Seth et al, J. Indian Chem.Soc., vol 88, 2011, pages 405-411 discloses studies on the synthesis of ammonium salts of 2,4-D to enhance its bioregulating potential. One of disadvantages of MCPA and 2,4-D is that, due to the presence of carboxyl group in their molecules, they can form under suitable pH conditions stable complexes with the species present in the soil, including metal ions, such as Hg, Co, Pb and Cd. This results in the change of properties of the active substance. Complexes of MCPA and 2,4-D with metal ions are insoluble and absorbed by the soil and their biodegradation is slower. Thus, the possibility of formation of complexes with metal is dangerous for the environment due to accumulation of these compounds in the plants and soil.

The solution of this problem proposed in the prior art were salts of the MCPA and 2,4-D anions formed with certain quaternary ammonium cations that have a melting point below 100 °C and belong to the group of chemical compounds called ionic liquids.

Ionic liquids are built of an organic or inorganic anion and an organic cation. A characteristic feature which allows to classify a compound to the group of ionic liquids is its melting point, which by definition cannot exceed 100 °C.

Quaternary ammonium salts, ionic liquids, comprising MCPA anion are disclosed in WO2008/140338. WO2008/140338 discloses, inter alia, ionic pairs of MCPA anion with methyltri(R)-, dimethyldi(R)-, and trimethyl(R)ammonium cations, where R can be a linear alkyl group containing 1 to 18 carbon atoms or a mixture of linear alkyl chains containing 1 to 20 carbon atoms (common names cocoalkyl or hydrogenated tallowalkyl), or polyoxyethylene group. Such ionic pairs are disclosed only generally, without exemplification of any specific compound having such structure. Ionic liquids comprising MCPA anion are also disclosed in J. Pernak et al., Tetrahedron 67(2011), 4838-4844. Among many others, disclosed are ionic pairs of MCPA anion with quaternary di(hydrogenated tallow alkyl)dimetyl and di(C₁₀H₂₁ alkyl)dimethyl ammonium cations, i.e. di(hydrogenated tallow)dimethylammonium (4-chloro-2-methylphenoxy)acetate and didecyldimethylammonium (4-chloro-2-methylphenoxy)acetate, respectively.

In WO2008/106118 there are described herbicidal ionic liquids formed in the reaction of 2,4-D in its acid form with equimolar amount of alkyl-N,N-dimethylamine or trialkylamine wherein the total number of carbon atoms is greater than 9.

Prior art ionic liquids have herbicidal activity due to the presence of MCPA or 2,4-D anion. Several further advantages of MCPA or 2,4-D anion containing ionic liquids are disclosed generally, i.e. reduced volatility and drift during and after application, thermal and chemical resistance, lack of reaction with metal ions due to the absence of free carboxylate group and resulting inhibition of the transport of a metal to the soil, limited tendency to deposit in natural environment and therefore more environmental friendliness comparing to the parent herbicides - MCPA or 2,4-D, as well as additional surfactant properties.

Despite of described advantages of known quaternary ammonium ionic liquids containing MCPA or 2,4-D anion, a need still exists of herbicidal compounds having high herbicidal activity.

Furthermore, a need still exits of novel herbicidal compounds having higher herbicidal activity than known compounds without loss of other advantageous properties of known compounds. This would allow to lower dosage of a herbicide and thus protect the environment.

A need also exists of novel herbicidal compounds that are more prone to biodegradation.

There is also a need of novel herbicides having consistence that makes their application easier.

There is also a need of novel herbicides that are less prone to acquiring resistance by controlled weeds.

These objects have been realized by novel ionic liquids - salt compounds of the invention comprising MCPA or 2,4-D anion.

### Summary of the invention

The present invention relates to a compound represented by the following general Formula (I) 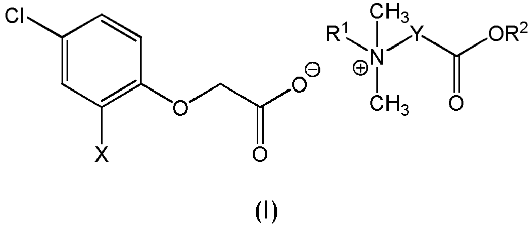 wherein
- X represents Cl or CH₃
- Y represents -CH₂- or -CH₂-CH(OH)-CH₂-,
- R¹ represents straight chain alkyl C1 to C18 or their mixtures, cocoamidopropyl, or straight chain C1-C18-alkylamidopropyl or their mixtures, and
- R² represents hydrogen atom or straight chain alkyl C1 to C18 or their mixtures.

One embodiment of the invention is the compound of the formula (I) wherein X represents Cl. This embodiment relates therefore to salts of 2,4-D herbicide.

One embodiment of the invention is the compound of the formula (I) wherein X represents CH₃. This embodiment relates therefore to salts of MCPA herbicide.

In another embodiment of the above compounds Y represents -CH₂-.

In further embodiment of the above compounds Y represents -CH₂-CH(OH)-CH₂-.

In one embodiment of the above compounds R¹ represents straight chain alkyl C1 to C18, such as C1, C2, C3, C4, C5, C6, C7, C8, C9, C10, C11, C12, C13, C14, C15, C16, C17 or C18.

Preferably, R¹ represents C1 alkyl. Also preferably, R¹ represents C12 (dodecyl) or C14 alkyl (tetradecyl).

In yet another embodiment of the above compounds R¹ represents a mixture of straight chain alkyls C1 to C18, such as for example a mixture of straight chain C12 alkyl (dodecyl) and C14 alkyl (tetradecyl).

In yet another embodiment of the above compounds R¹ represents cocoamidopropyl.

In further embodiment of the above compounds R¹ represents straight chain C1-C18-alkylamidopropyl or their mixture.

In one embodiment of the above compounds R² represents hydrogen atom.

In another embodiment of the above compounds R² represents straight chain alkyl C1 to C18, such as C1, C2, C3, C4, C5, C6, C7, C8, C9, C10, C11, C12, C13, C14, C15, C16, C17 or C18, especially straight chain C12 alkyl (dodecyl).

In one specific embodiment of the above compounds X represents Cl, Y represents -CH₂-, R¹ represents C1 alkyl, and R² represents H.

In another specific embodiment of the above salts X represents Cl, Y represents -CH₂-CH(OH)-CH₂-, R¹ represents C1 alkyl, and R² represents H.

In one specific embodiment of the above compounds X represents CH₃, Y represents -CH₂-, R¹ represents C1 alkyl, and R² represents H.

In another specific embodiment of the above salts X represents CH₃, Y represents -CH₂-CH(OH)-CH₂-, R¹ represents C1 alkyl, and R² represents H.

The term "coco" or "cocoalkyl" is commonly known in the field and relates to alkyl groups of coconut oil fatty acids. It is generally recognized that "coco" or "cocoalkyl" typically is a mixture of saturated and unsaturated alkyl groups C8, C10, C12, C14, C16, and C18, the percentage of groups with double bonds is from 1 to 6%, and the percentage of C12 group is from 45 to 55%.

Herbicidally active ionic liquids of the formula (I) of the invention are quaternary ammonium salts formed with carboxybetaine type cations.

According to the invention, carboxybetaine type cations include N,N,N-tri-methylglycine cation (CH₃)₃N⁺CH₂CO₂H for which in formula (I) Y represents -CH₂- and R² represents hydrogen atom (hereinafter simply called betaine - Bet), and its derivatives wherein R² represents straight chain alkyl C1 to C18 or their mixtures. Carboxybetaine type cations include also betaine derivatives, wherein one methyl group in N,N,N-trimethylglycine cation is replaced by straight chain alkyl up to C18 or their mixtures, cocoamidopropyl, or straight chain C1-C18-alkylamidopropyl or their mixtures, and R² represents hydrogen atom or straight chain alkyl C1 to C18 or their mixtures.

According to the invention, carboxybetaine type cations include also carnitine cation (CH₃)₃N⁺-CH₂-CH(OH)-CH₂-COOH wherein in formula (I) Y represents -CH₂-CH(OH)-CH₂- and R² represents hydrogen atom (designated hereinafter simply as carnitine - Car), and its derivatives wherein R² represents straight chain alkyl C1 to C18 or their mixtures. Carboxybetaine type cations include also carnitine derivatives, wherein one methyl group in carnitine cation is replaced by straight chain alkyl up to C18 or their mixtures, cocoamidopropyl, or straight chain C1-C18-alkylamidopropyl or their mixtures, and R² represents hydrogen atom or straight chain alkyl C1 to C18 or their mixtures.

Carboxybetaine type cations are derived from aminoacids, can be easy manufactured, have low toxicity and are completely biodegradable. Due to the zwitterionic structure and presence of various alkyl groups betaines possess biocidic properties against fungi, viruses and bacteria, both Gram-positive and Gram-negative. They are considered environmentally friendly and are widely used in many fields. Betaines with long alkyl chain can be easily commercially manufactured by quaternization of respective fatty amines.

Exemplary ionic liquids of the invention having betaine type cation and (2,4-dichlorophenoxy)acetate or (4-chloro-2-methylphenoxy)acetate anion are:
- betaine (2,4-dichlorophenoxy)acetate (X = Cl, Y = -CH₂-, R¹ = CH₃, R² = H)
- betaine (4-chloro-2-methylphenoxy)acetate (X = CH₃, Y = -CH₂-, R¹ = CH₃, R² = H)
- carnitine (2,4-dichlorophenoxy)acetate (X = Cl, Y = -CH₂-CH(OH)-CH₂-, R¹ = CH₃, R² = H)
- carnitine (4-chloro-2-methylphenoxy)acetate (X = CH₃, Y = -CH₂-CH(OH)-CH₂-, R¹ = CH₃, R² = H)
- dodecyldimethylglycine (2,4-dichlorophenoxy)acetate (X = Cl, Y = -CH₂-, R¹ = C₁₂H₂₅, R² = H)
- dodecyldimethylglycine (4-chloro-2-methylphenoxy)acetate (X = CH₃, Y = - CH₂-, R¹ = C₁₂H₂₅, R² = H)
- tetradecyldimethylglycine (4-chloro-2-methylphenoxy)acetate (X = CH₃, Y = -CH₂-, R¹ = C₁₄H₂₉, R² = H)
- (cocoamidopropyl)dimethylglycine (2,4-dichlorophenoxy)acetate (X = Cl, Y = -CH₂-, R¹ = cocoamidopropyl, R² = H)
- (cocoamidopropyl)dimethylglycine (4-chloro-2-methylphenoxy)acetate (X = CH₃, Y = -CH₂-, R¹ = cocoamidopropyl, R² = H)
- dodecanoylbetaine (2,4-dichlorophenoxy)acetate (X = Cl, Y = -CH₂-, R¹ = CH₃, R² = C₁₂H₂₅)
- dodecanoylbetaine (4-chloro-2-methylphenoxy)acetate (X = CH₃, Y = -CH₂-, R¹ = CH₃, R² = C₁₂H₂₅)
- dodecanoylcarnitine (4-chloro-2-methylphenoxy)acetate (X = CH₃, Y = -CH₂-CH(OH)-CH₂-, R¹ = CH₃, R² = C₁₂H₂₅).

Novel compounds of the invention have herbicidal activity due to the presence of (4-chloro-2-methylphenoxy)acetate or (2,4-dichlorophenoxy)acetate anion, and have advantageous properties, namely:
- due to low melting point below 100°C are ionic liquids,
- have high thermal and chemical stability,
- are non-volatile - their vapor pressure at ambient temperature is in practice non-measurable,
- can be hydrophobic or hydrophilic, depending on the type of substituents in the quaternary ammonium cation,
- due to the presence of a cation with long alkyl substituent are cationic surfactants,
- do not form complexes with heavy metals, which results in inhibition of the transport of a heavy metal to the protected plant,
- due to the presence of large cation possess also bactericidal and fungicidal properties, and can be considered multifunctional compounds,
- due to the presence of ester or amide group in the cation and natural origin are characterized by high level of biodegradation.

It is also believed that the ionic liquids of the invention show little toxicity to warm-blooded animals.

Furthermore, the presence of betaine type cation diminishes toxicity of 2,4-D and MCPA anion with respect to fish and daphnia, thus reducing ecotoxicity of these herbicides.

Herbicidal activity of ionic liquids is enhanced if long alkyl chain is present in the cation.

Novel compounds of the formula (I) are herbicidally active ionic liquids and can find use for combatting and controlling unwanted vegetation, such as weeds.

The object of the invention is therefore a herbicidal composition, comprising as herbicidally active ingredient a compound of the formula (I) as defined above.

Typically, the composition of the invention will be a formulation containing the compound of the invention in a solution in water, organic solvent, such as methanol or isopropanol, or their mixture. Depending on the concentration of the active substance, the formulation can be a ready for use formulation or concentrate for dilution prior to final use.

Further to the compound of the formula (I), herbicidal composition formulation will contain typical auxiliary agents used in agrochemical compositions. In particular, wetting agents and agents improving stability of the formulation, such as thickener, antifoaming agent, homogenizer or their mixtures, can be included.

In addition to the herbicidally active ionic liquid of the formula (I) of the invention, the herbicidal composition may include second herbicidally active ingredient, another than anion present in the ionic liquid. Said second herbicidally active ingredient may be selected from herbicidally active carboxylic acids, phosphonic acids and their mixtures. Examples of herbicidally active carboxylic acids are phenoxycarboxylic acids, such as 2,4-D, MCPA, dicamba, and MCPP. Examples of herbicidally active phosphonic acids are glyphosate and its derivatives.

### Preparation of ionic liquids of the invention.

Compounds of the general formula (I) as defined above can be prepared by reaction of anion exchange of halogenide of the general formula (II) 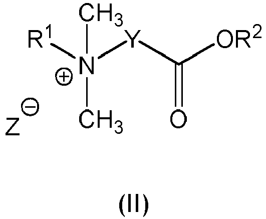 wherein R¹, R² and Y have the meaning as defined above for the general formula (I), and Z is Cl, Br or I,
with sodium, potassium, lithium or ammonium salt of (2,4-dichlorophenoxy)-acetic or (4-chloro-2-methylphenoxy)acetic acid, respectively.

The reaction is carried out at the molar ratio of halogenide (II) to the salt of respective acid from 1:0.95 to 1:1.05, preferably 1:1.

Temperature of the reaction is from 0 to 100 °C, preferably 20 °C.

The reaction is performed in water or in an organic solvent or their mixtures. Organic solvent can be selected from methanol, ethanol, propanol, isopropanol, and butanol.

Preferably, the solution of (2,4-dichlorophenoxy)acetic acid or (4-chloro-2-methylphenoxy)acetic acid salt is added to halogenide of the formula (II) placed in the reaction vessel.

Preferably, when reaction is carried out in water, aqueous solution of (2,4-dichlorophenoxy)acetic acid or (4-chloro-2-methylphenoxy)acetic acid salt is added to aqueous solution of halogenide of the formula (II).

Preferably, when reaction is carried out in organic solvent, the solution of (2,4-dichlorophenoxy)acetic or (4-chloro-2-methylphenoxy)acetic acid salt in the organic solvent is added to halogenide of the formula (II) or the solution of halogenide of the formula (II) in an organic solvent. Alternatively, solid (2,4-dichlorophenoxy)acetic or (4-chloro-2-methylphenoxy)acetic acid salt is added to the solution of halogenide of the formula (II) in an organic solvent.

After completion of the reaction, its product, the compound of the formula (I) can be isolated from the reaction medium in solid form.

Isolation of the compound of the formula (I) from aqueous reaction medium can be performed by two-phase extraction with an organic solvent such as chloroform, dichloromethane, toluene or ethyl acetate, separation of the organic phase, removal of the solvent to obtain solid product of the formula (I), and drying the product.

Isolation of the compound of the formula (I) from aqueous reaction medium can be also performed by evaporation of water, addition of an organic solvent such as chloroform, dichloromethane, toluene or ethyl acetate to precipitate inorganic side-product, filtration of the inorganic side-product, removal of the solvent from the filtrate to obtain solid product of the formula (I), and drying the product.

Isolation of the compound of the formula (I) from organic reaction medium can be performed by filtration of inorganic side-product, removal of the solvent from the filtrate to obtain solid product of the formula (I), and drying the product.

To obtain compound of the general formula (I) as a solution, the reaction of anion exchange between halogenide of the formula (II) and sodium, potassium, lithium or ammonium salt of (2,4-dichlorophenoxy)acetic acid or (4-chloro-2-methylphenoxy)acetic acid, as described above, can be performed in a mixture of water with an organic solvent selected from the group consisting of methanol, ethanol, propanol, isopropanol, and butanol, or a mixture of two or more organic solvents having molar weight below 100 g/mol. In such a case, preferable halogenide of the formula (II) is used as a solution in an organic solvent, and salt of (2,4-dichlorophenoxy)acetic acid or (4-chloro-2-methylphenoxy)acetic acid as an aqueous solution.

To obtain herbicidal composition of the invention comprising compound of the general formula (I) as defined above as a herbicidal ionic liquid and another second herbicidally active compound which is not in the form of a ionic liquid, the compound of the general formula (I) is added to the solution of sodium, potassium, lithium or ammonium salt, or an ester or the second herbicidally active compound in water or in a mixture of water with an organic solvent selected from the group consisting of methanol, ethanol, propanol, isopropanol, and butanol.

Furthermore, the herbicidal composition of the invention can include auxiliary substances typically used in herbicidal formulations, such as wetting agents and stabilizers of the final formulation, such as thickeners, anti-foaming agents, homogenizers or their mixtures. Such auxiliary substances are known for a person skilled in the art and can be easily selected.

The invention is further exemplified by its embodiments presented in the below Examples.

The structures of the compounds prepared in the Examples were confirmed by proton and carbon nuclear magnetic resonance.

In the Examples below betaine (Bet) refers to unsubstituted N,N,N-trimethyl-glycine and carnitine (Car) refers to unsubstituted carnitine.

### Example 1

### Betaine (2,4-dichlorophenoxy)acetate - [Bet][2,4-D]

15.4 g (0.10 mol) of betaine hydrochloride were placed in a round-bottom flask equipped with a magnetic stirrer. Subsequently, to the flask were added 31.1 g (0.12 mol) of potassium (2,4-dichlorophenoxy)acetate dissolved in 50 cm³ of boiling metanol. After carrying out the reaction at room temperature for 45 minutes, the reaction mixture was placed for a period of 15 minutes at -20 °C. Solid potassium chloride that precipitated was filtered off, and then methanol was evaporated from the filtrate in a rotary vacuum evaporator to obtain the title product. The product was dried in a vacuum drier for 24 hours at 50 °C. The yield was 92%.
¹H NMR (300 MHz, CD₃OD) δ ppm = 3.27 (s, 9H); 3.93 (s, 2H); 4.73 (s, 2H); 6.95 (d, *J* = 8.8 Hz, 1H); 7.21 (d, *J* = 2.5 Hz, 1H); 7.40 (s, 1H); ¹³C NMR (75 MHz, CD₃OD) δ ppm = 172.2; 168.7; 154.1; 130.8; 128.7; 127.2; 124.6; 115.8; 67.0; 66.6; 53.9.
Elemental analysis CHN for C₁₃H₁₇Cl₂NO₅ (Mₘₒₗ = 338.1 g mol⁻¹): calculated (%): C = 46.17; H = 5.07; N = 4.14; measured: C = 46.49; H = 5.22; N = 3.89.

### Example 2

### Betaine (4-chloro-2-methylphenoxy)acetate - [Bet][MCPA]

9.9 g (0.05 mol) of betaine bromohydride were dissolved in 30 cm³ of ethanol. Then stoichiometric amount of sodium (4-chloro-2-methylphenoxy)acetate (0.05 mol) dissolved in 50 cm³ of ethanol was added in small portions while stirring. The reaction of anion exchange was carried out at 40 °C for 60 minutes, and then precipitated solid sodium bromide was filtered-off and ethanol was evaporated from the filtrate in a rotary vacuum evaporator to obtain the title product. The product was dried in a vacuum drier for 24 hours at 50 °C. The yield was 97%. ¹H NMR (300 MHz, CD₃OD) δ ppm = 2.23 (s, 3H); 3.25 (s, 9H); 3.89 (s, 2H); 4.63 (s, 2H); 6.74 (d, *J* = 8.7 Hz, 1H); 7.09 (d, *J* = 3.3 Hz, 1H); 7.11 (s, 1H); ¹³C NMR (75 MHz, CD₃OD) δ ppm = 173.1; 168.7; 156,4; 131.4; 130.3; 127.4; 126.7; 113.6; 66.9; 66.6; 53.9; 16.4. Elemental analysis CHN for C₁₄H₂₀ClNO₅ (Mₘₒₗ = 317.8 g mol⁻¹) calculated (%): C = 52.92; H = 6.34; N = 4.41; measured: C = 52.77; H = 6.16; N = 4.72.

### Example 3

### Carnitine (2,4-dichlorophenoxy)acetate - [Car][2,4-D]

19.8 g (0.10 mol) of carnitine hydrochloride were placed in a reaction vessel. Then 50 cm³ of propanol and stoichiometric amount of (2,4-dichlorophenoxy)-acetic acid lithium salt (0.08 mol). The reaction mixture was stirred for 2 hours at 60 °C. Precipitated solid lithium chloride was filtered-off after cooling of the reaction mixture to 25 °C and the solvent was evaporated from the filtrate. Then the product was dried for 24 hours at 50 °C under reduced pressure. Carnitine (2,4-dichlorophenoxy)acetate was obtained with the yield of 91%.
¹H NMR (300 MHz, CD₃OD) δ ppm = 2.48 (d, *J* = 6.5 Hz, 2H); 3.23 (s, 9H); 3.,43 (m, 2H); 4.54 (m, 3H); 6.93 (d, *J* = 8.6 Hz, 1H); 7.22 (d, *J* = 2.6 Hz, 1H); 7.39 (s, 1H); ¹³C NMR (75 MHz, CD₃OD) δ ppm =175.4; 174.7; 154.8; 130.7; 128.7; 126.6; 124.6; 115.9; 71.5; 68.9; 64.7; 55.0; 42.0.
Elemental analysis CHN for C₁₅H₂₁Cl₂NO₆ (Mₘₒₗ = 382.2 g mol⁻¹): calculated (%): C = 47.13; H = 5,54; N = 3.66; measured: C = 46.88; H = 5.75 N = 3.91.

### Example 4

### Carnitine (4-chloro-2-methylphenoxy)acetate - [Car][MCPA]

To 92.5 g of 25% butanol solution of carnitine hydroiodide (0.08 mol) placed in a reaction vessel were added in small portions while stirring 21.5 g (0.09 mol) of (4-chloro-2-methylphenoxy)acetic potassium salt. Stirring was continued for 30 minutes at room temperature, then precipitated solid potassium iodide was filtered-off and methanol evaporated from the filtrate using rotary vacuum evaporator to obtain the title product. Product was dried in vacuum for 24 hours at 50 °C. The yield was 96%.
¹H NMR (300 MHz, CD₃OD) δ ppm = 2.24 (s, 3H); 2.49 (d, *J* = 6.5 Hz, 2H); 3.20 (s, 9H); 3.42 (m, 2H); 4.49 (s, 2H); 4.53 (m, 1H); 6.73 (d, *J* = 8.6 Hz, 1H); 7.07 (dd, *J_{1,2}* = 8.6 Hz, *J_{1,3}* = 2.7 Hz, 1H) 7.11 (d, *J* = 2.4 Hz, 1H); ¹³C NMR (75 MHz, CD₃OD) δ ppm = 175.3; 156.9; 131.2; 130.2; 127.3; 126.1; 113.6; 71.4; 68.2; 64.6; 54.9; 42.0; 16.5.
Elemental analysis CHN for C₁₆H₂₄ClNO₆ (Mₘₒₗ = 361.8 g mol⁻¹) calculated (%): C = 53.11; H = 6.69; N = 3.87; measured: C = 53.34; H = 6.47 N = 3.60.

### Example 5

### Dodecyldimethylglycine (2,4-dichlorophenoxy)acetate - [C₁₂Bet][2,4-D]

30.8 g of dodecyldimethylglycine hydrochloride (0.10 mol) were placed in a reaction flask and dissolved in 75 cm³ of distilled water. To thus obtained solution stoichiometric amount of (2,4-dichlorophenoxy)acetic sodium salt (0.10 mol) dissolved in 50 cm³ of distilled water was added in small portions while stirring. Stirring was continued for 20 minutes at 30 °C, and then the product was extracted from the mixture with chloroform. Organic phase was separated, washed 4 times with distilled water, and then solvent was evaporated using rotary vacuum evaporator to obtain the title product. The product was dried for 24 hours at 50 °C under reduced pressure. The yield was 92%.
¹H NMR (300 MHz, DMSO-*d₆*) δ ppm = 0.86 (t, *J* = 6.6 Hz, 3H); 1.24 (m, 18H); 1.64 (s, 2H); 3.15 (s, 6H); 3.52 (m, 2H); 3.77 (s, 2H); 4.73 (s, 2H); 7.04 (d, *J* = 9.0 Hz, 1H); 7.31 (dd, *J_{1,2}* = 8.9 Hz, *J_{1,3}* = 2.6 Hz, 1H); 7.51 (d, *J* = 2.7 Hz, 1H); ¹³C NMR (75 MHz, DMSO-*d₆*) δ ppm = 169.6; 165.5; 152.7; 129.2; 127.8; 124.4; 122.2; 114.9; 65.9; 63.3; 63.1; 50.1; 31.4; 29.1; 22.2; 13.9. Elemental analysis CHN for C₂₄H₃₉Cl₂NO₅ (Mₘₒₗ = 492.5 g mol⁻¹) calculated (%): C = 58.53; H = 7.98; N = 2.84; measured: C = 58.85; H = 8.30; N = 2.64.

### Example 6

### Dodecyldimethylglycine (4-chloro-2-methylphenoxy)acetate - [C₁₂Bet][MCPA]

30.8 g of 50% w/v aqueous solution dodecyldimethylglycine hydrochloride (0.05 mol) were placed in a reaction vessel equipped with a magnetic stirrer. Stoichiometric amount of (4-chloro-2-methylphenoxy)acetic potassium salt (0.05 mol) dissolved in 30 cm³ of distilled water was added in small portions while stirring. Stirring was continued for 15 minutes at ambient temperature and then water was evaporated and 100 cm³ of acetone added. Potassium chloride precipitated from the solution was filtered-off and solvent evaporated from filtrate to obtain the title product. The product was dried under vacuum for 24 hours at 50 °C. The yield was 97%.
¹H NMR (300 MHz, DMSO-*d₆*) δ ppm = 0.86 (t, *J* = 6.7 Hz, 3H); 1.24 (m, 18H); 1.61 (s, 2H); 2.18 (s, 3H); 3.12 (s, 6H); 3.48 (m, 2H); 3.67 (s, 2H); 4.56 (s, 2H); 6.78 (d, *J* = 8.7 Hz, 1H); 7.11 (dd, *J_{1,2}* = 8.7 Hz, *J_{1,3}* = 2.7 Hz, 1H); 7.18 (d, *J* = 2.7 Hz, 1H); ¹³C NMR (75 MHz, DMSO-*d₆*) δ ppm = 170.4; 165.2; 155.2; 129.8; 128.2; 126.1; 123.7; 112.8; 65.9; 63.8; 62.9; 49.9; 31.3; 29.1; 22.1; 15.9; 13.9. Elemental analysis CHN for C₂₅H₄₂ClNO₅ (Mₘₒₗ = 472.1 g mol⁻¹) calculated (%): C = 63.61; H = 8.97; N = 2,97; measured: C = 63.89; H = 8.70; N = 3.20.

### Example 7

### C₁₂-C₁₄-Alkyldimethylglycine (4-chloro-2-methylphenoxy)acetate - [C₁₂-C₁₄Bet][MCPA]

A mixture of 45.2 g of 50% aqueous solution of dodecyldimethylglycine hydrochloride (0.07 mol) and 26.3 g of 40% w/v aqueous solution of tetradecyldimethylglycine hydrochloride (0.03 mol) was placed in a reaction flask equipped with a magnetic stirrer, then stoichiometric amount of (4-chloro-2-methyl-phenoxy)acetic potassium salt (0.1 mol) dissolved in 60 cm³ of distilled water was added in small portions while stirring. Stirring was continued for 30 minutes at ambient temperature, then water was evaporated and 150 cm³ of anhydrous methanol was added. Potassium chloride precipitated from the solution was filtered-off, and solvent was evaporated from the filtrate to obtain the title product. The product was dried under vacuum for 24 hours at 50 °C. The yield was 95%.
¹H NMR (300 MHz, DMSO-d₆) δ ppm = 0.88 (t, *J* = 6.7 Hz, 3H); 1.26 (m, 22H); 1.73 (s, 2H); 2.15 (s, 3H) 3.24 (m, 2H); 3.30 (s, 6H); 4.18 (s, 2H); 5.05 (s, 2H); 6.81 (d, *J* = 8.7 Hz, 1H); 7.19 (dd, *J_{1,2}* = 8.7 Hz, *J_{1,3}* = 2.7 Hz, 1H); 7.32 (d, *J* = 2.7 Hz, 1H), 11.0 (s, 1H). ¹³C NMR (75 MHz, DMSO-d₆) δ ppm = 180.5; 168.2; 156.7; 129.8; 127.9; 126.5; 125.9; 111.8; 78.3; 64.1; 62.4; 49.9 [2]; 31.9; 29.6 [8]; 26.8; 25.1; 22.7; 14.9; 14.1. Elemental analysis CHN for C_{26,6}H_{38,4}ClNO₅ (Mₘₒₗ = 487.33 g mol⁻¹) calculated (%): C = 65.65; H = 7.88; N = 2.87; measured: C = 64.90; H = 7.72; N = 2.80.

### Example 8

### (Cocoamidopropyl)dimethylglycine (2,4-dichlorophenoxy)acetate - [CAPBet][2,4-D]

26.3 g of (cocoamidopropyl)dimethylglycine hydrobromide (0.06 mol) and 50 cm³ of distilled water were placed in a flask and the reaction mixture was stirred until a clear solution was obtained. Then stoichiometric amount of (4-chloro-2-methylphenoxy)acetic acid lithium salt (0.06 mol) dissolved in 40 cm³ of distilled water was added in small portions while stirring. Stirring was continued for 40 minutes at ambient temperature, then water was evaporated and 100 cm³ of acetonitrile were added. Lithium bromide precipitated from the solution was filtered-off, then solvent was evaporated from the filtrate to obtain the title product. The product was dried under vacuum for 24 hours at 50 °C. The yield was 93%.
¹H NMR (400 MHz, DMSO-*d₆*) δ ppm = 0.86 (t, *J* = 6.7 Hz, 3H); 1.24 (m, 16H); 1.49 (m, 2H); 1.82 (m, 2H); 2.08 (t, *J* = 7.5 Hz, 2H); 3.10 (m, 2H); 3.19 (s, 6H); 3.54 (m, 2H); 4.18 (s, 2H); 4.69 (s, 2H); 7.09 (d, *J* = 8.8 Hz, 1H); 7.35 (dd, *J_{1,2}* = 8,9 Hz, *J_{1,3}* = 2.7 Hz, 1H); 7.46 (d, *J* = 2.7 Hz, 1H); 8.17 (t, *J* = 5.7 Hz, 1H); ¹³C NMR (100 MHz, DMSO-*d₆*) δ ppm = 172.5; 169.7; 166.1; 152.9; 129.4; 127.9; 124.5; 122.2; 114.7; 65.5; 62.5; 61.9; 50.7; 35.7; 31.3; 29.2; 28.8; 25.3; 22.8; 22.1; 13.9.

### Example 9

### (Cocoamidopropyl)dimethylglycine (4-chloro-2-methylphenoxy)acetate - [CAPBet][MCPA]

To the reaction vessel were added 33.7 g of 35% w/v aqueous solution of (cocoamidopropyl)dimethylglycine hydrochloride (0.03 mol) and then stoichiometric amount of (4-chloro-2-methylphenoxy)acetic sodium salt (0.03 mol) dissolved in 30 cm³ of distilled water. The mixture was stirred for 20 minutes at 30 °C, and then was extracted with dichloromethane. Organic phase was separated, washed 4 times with distilled water and then solvent was evaporated using rotary vacuum evaporator to obtain the title product. The product was dried for 24 hours at 50 °C under reduced pressure. The yield was 95%.
¹H NMR (400 MHz, DMSO-*d₆*) δ ppm = 0.86 (t, *J* = 6.7 Hz, 3H); 1.23 (m, 16H); 1.49 (t, *J* = 6.8 Hz, 2H); 1.82 (m, 2H); 2.08 (t, *J* = 7.5 Hz, 2H); 2.19 (s, 3H); 3.10 (kw, *J* = 6.1 Hz, 2H); 3.19 (s, 6H); 3.54 (m, 2H); 4.03 (s, 2H); 4.70 (s, 2H); 6.84 (d, *J* = 8.7 Hz, 1H); 7.16 (dd, *J_{1,2}* = 8.8 Hz, *J_{1,3}* = 2.6 Hz, 1H); 7.18 (d, *J* = 2.7 Hz, 1H); 8.11 (t, *J* = 5.6 Hz, 1H); ¹³C NMR (100 MHz, DMSO-*d₆*) δ ppm = 172.5; 170.0; 166.1; 154.9; 130.0; 128.4; 126.3; 124.2; 112.9; 65.1; 62.3; 61.8; 50.4; 35.5; 31.4; 29.2; 28.8; 25.3; 22.8; 22.2; 15.8; 14.0.

### Example 10

### Dodecanoylbetaine (2,4-dichlorophenoxy)acetate - [Bet-OC₁₂][2,4-D]

In a round-bottom flask equipped with magnetic stirrer 32.2 g (0.10 mol) of dodecanoylbetaine chloride were placed. Then 31.1 g (0.12 mol) of sodium (2,4-dichlorophenoxy)acetate dissolved in 50 cm³ of boiling isopropanol were added. The anion exchange reaction was carried out at room temperature for 2 hours and then the reaction mixture was placed for 15 minutes at -20 °C. Precipitated sodium chloride was filtered-off and isopropanol was evaporated from the filtrate using rotary vacuum evaporator to obtain the title product. The product was dried in a vacuum drier for 24 hours at 50 °C. The yield was 91%.
¹H NMR (300 MHz, DMSO-*d₆*) δ ppm = 0.86 (m, 3H); 1.24 (m, 18H); 2.23 (m, 2H); 3.15 (s, 9H); 3.58 (s, 2H); 4.07 (m, 2H); 4.36 (s, 2H); 6.70 (d, *J* = 9.0 Hz, 1H); 7.25 (d, *J* = 9.0 Hz, 1H); 7.48 (s, 1H);
¹³C NMR (75 MHz, DMSO-*d₆*) δ ppm = 172.3; 169.4; 153.5; 128.8; 127.6; 123.3; 121.,8; 115.0; 67.9; 60.7; 53.1; 44.5; 32.6; 31.3; 29.2; 29.1; 29.0; 28.8; 28.2; 25.6; 22.1; 13.9.
Elemental analysis CHN for C₂₅H₄₁Cl₂NO₅ (Mₘₒₗ = 506.5 g mol⁻¹) calculated (%): C = 59.28; H = 8.16; N = 2.77; measured: C = 59.51; H = 7.84; N = 2.53.

### Example 11

### Dodecanoylbetaine (4-chloro-2-methylphenoxy)acetate - [Bet-OC₁₂][MCPA]

32.2 g of 50% w/v aqueous solution of dodecanoylbetaine chloride (0.05 mol) were placed in a reaction vessel equipped with a magnetic stirrer and then stoichiometric amount of (4-chloro-2-methylphenoxy)acetic potassium salt (0.05 mol) dissolved in 40 cm³ of distilled water was added in small portions while stirring. Stirring was continued for 30 minutes at ambient temperature, then water was evaporated and 75 cm³ ethanol were added. Potassium chloride precipitated from the solution was filtered-off, and solvent was evaporated from filtrate to obtain the title product. The product was dried in vacuum for 24 hours at 50 °C. The yield was 93%.
¹H NMR (400 MHz, DMSO-*d₆*) δ ppm = 0.85 (t, *J* = 6.8 Hz, 3H); 1.23 (m, 18H); 1.59 (m, 2H); 2.16 (s, 3H); 3.25 (s, 9H); 4.15 (t, *J* = 6.2 Hz, 2H); 4.32 (s, 2H); 4.56 (s, 2H); 6.72 (d, *J* = 8.8 Hz, 1H); 7.10 (dd, *J_{1,2}* = 8.8 Hz, *J_{1,3}* = 2.8 Hz, 1H); 7.14 (d, *J* = 2,8 Hz, 1H). ¹³C NMR (100 MHz, DMSO-*d₆*) δ ppm = 175.1; 165.1; 155.8; 129.5; 127.0; 126.0; 112.8; 67.4; 65.7; 62.3; 53.0; 31.4; 29.1; 29.0; 28.8; 28.7; 27.9; 25.3; 22.2; 16.0; 14.0. Elemental analysis CHN for C₂₆H₄₄ClNO₅ (Mₘₒₗ = 486.1 g mol⁻¹) calculated (%): C = 64.24; H = 9.12; N = 2.88; measured: C = 64.05; H = 9.39; N = 3.12.

### Example 12

### Dodecanoylcarnitine (4-chloro-2-methylphenoxy)acetate - [Car-OC₁₂] [MCPA]

To the flask were added 68.6 g of 40% w/v aqueous solution of dodecanoylcarnitine iodide (0.06 mol) and then stoichiometric amount of (4-chloro-2-methylphenoxy)acetic lithium salt (0.06 mol) dissolved in 50 cm³ of distilled water. The mixture was stirred for 30 minutes at room temperature, and then the product was extracted with toluene. Organic phase was separated, washed 4 times with distilled water, and solvent was evaporated rotary vacuum evaporator to obtain the title product. The product was dried for 24 hours at 50 °C under reduced pressure. The yield was 92%.
¹H NMR (300 MHz, DMSO-*d₆*) δ ppm = 0.86 (t, *J* = 6.9 Hz, 3H); 1.23 (m, 18H); 1.56 (m, 2H); 2.15 (s, 3H); 2.48 (d, *J* = 6.6 Hz, 2H); 3.15 (s, 9H); 3.37 (m, 1H); 3.70 (m, 1H); 4.00 (m, 2H); 4.18 (s, 2H); 6.70 (d, *J* = 8.7 Hz, 1H); 7.09 (dd, *J_{1,2}* = 8.7 Hz, *J_{1,3}* = 3.0 Hz, 1H) 7.12 (d, *J* = 2.4 Hz, 1H); ¹³C NMR (75 MHz, DMSO-*d₆*) δ ppm = 170.4; 156.2; 129.4; 127.8; 125.9; 122.6; 112.9; 68.2; 64.1; 62.1; 53.3; 40.6; 31.3; 29.1; 29.0; 28.8; 28.1; 25.4; 22.1; 16.0; 13.9.
Elemental analysis CHN for C₂₈H₄₈ClNO₆ (Mₘₒₗ = 530.1 g mol⁻¹) calculated (%): C = 63.44; H = 9.13; N = 2.65; measured: C = 63.07; H = 9.50; N = 2.89.

### Example 13

### Solution of dodecyldimethylglycine (4-chloro-2-methylphenoxy)acetate - [C₁₂Bet][MCPA]

348.1 g of 11.5% w/v isopropanol solution of dodecyldimethylglycine hydroiodide (0.10 mol) were placed in the flask and stoichiometric amount of (4-chloro-2-methylphenoxy)acetic potassium salt (0.10 mol) dissolved in 100 cm³ of distilled water was added in small portions while stirring. Stirring was continued for 15 minutes at ambient temperature to obtain ready solution of herbicidal ionic liquid.

Two-phase titration performed in accordance with the standard PN-EN ISO 2871 - 1:2000 showed the content of cationically active compound, i.e. [C₁₂Bet][MCPA] at 96% (corresponds to concentration of herbicidal ionic liquid in the solution of 9.6%).

### Example 14

### Herbicidal composition C₁₂-C₁₄-alkyldimethylglycine (4-chloro-2-methyl-phenoxy)acetate:dicamba - [C₁₂-C₁₄Bet][MCPA]:dikamba

To the reactor of working volume 10 dm³ equipped with mechanical stirrer and heating jacket were introduced 3000 cm³ of water and 24.6 g of solid NaOH. To the obtained solution were added in three portions 140 g of solid dicamba. Complete homogenization of the reaction mixture was reached at pH = 8.5. To the thus obtained aqueous solution of dicamba sodium salt 600 cm³ of isopropanol were added and 2557 g of 95% C₁₂-C₁₄alkyldimethylglycine (4-chloro-2-methylphenoxy)acetate heated to 65 °C were added dropwise. Reaction mixture was heated at 70 °C for 1 hour. After complete homogenization the product was cooled to room temperature. 6193 g of the water-isopropanol solution were obtained.

HPLC analysis of the content of the active substance in the product showed the content of dicamba of 2.15% and C₁₂-C₁₄-alkyldimethylglycine (4-chloro-2-methylphenoxy)acetate of 39.22%.

### The use of ionic liquids of the invention as herbicides

### Tests of herbicidal activity under greenhouse conditions

Test plants were white mustard (*Sinapis alba*), common lambsquarters *(Chenopodium album),* cornflower (*Centaurea cyanus*) and field penny-cress (*Thlaspi arvense*). Weed seeds were sown to the depth of 1 cm to plastic pots filled with peat-based potting material. After formation of cotyledons plants were thinned to four plants in each pot. Tested compounds were applied by spraying plants at the growth stage of four leaves. The application was performed by cabin sprayer equipped with Tee Jet 1102 flat-fan nozzle, that moved with a constant speed of 3.1 m/s on the height of 40 cm from plant tips. The operating pressure was 0.2 MPa, and spray volume calculated per 1 ha was 200 dm³.

Betaine (4-chloro-2-methylphenoxy)acetate [Bet][MCPA], betaine (2,4-dichloro-phenoxy)acetate [Bet][2,4-D], carnitine (4-chloro-2-methylphenoxy)acetate [Car][MCPA] and carnitine (2,4-dichlorophenoxy)acetate [Car][2,4-D] were dissolved in 200 dm³ of water:isopropanol (1:1 v/v) mixture in the amount corresponding to 400 g of (4-chloro-2-methylphenoxy)acetate (MCPA) or (2,4-dichlorophenoxy)acetate (2,4-D) anions per 1 ha, this corresponding to the concentration of 0.2%, and tested for efficacy against white mustard (*Sinapis alba*) and common lambsquarters (*Chenopodium album*) plants (Table 1). In the second experiment, dodecyldimethylglycine (4-chloro-2-methylphenoxy)acetate [C₁₂Bet][MCPA], or dodecyldimethylglycine (2,4-dichlorophenoxy)acetate [C₁₂Bet][2,4-D] were dissolved in 200 dm³ of water:isopropanol (1:1 v/v) mixture in the amount corresponding to 400 g of (4-chloro-2-methylphenoxy)acetate (MCPA) or (2,4-dichlorophenoxy)acetate (2,4-D) anions per 1 ha, this corresponding to concentration of 0.2% w/v, and tested for efficacy against common lambsquarters (*Chenopodium album*) and cornflower (*Centaurea cyanus*) plants (Table 2). Commercial herbicides containing 2,4-D (600 g of the active substance as dimethylammonium salt in 1 dm³) designated as Herbicide X; and MCPA (300 g of the active substance as sodium-potassium salt in 1 dm³) designated as Herbicide Y were applied as the reference products. After application plants were again placed in the greenhouse at 20 °C and air humidity 60%. Two weeks after application plants were cut just beneath the soil and their fresh mass was determined with a accuracy of 0.01 g, separately for each pot. Test was performed in 4 replicates in a fully random layout. Data were expressed as percent of fresh weight reduction compared with untreated plants.

Obtained results are presented in Tables 1 and 2 as percent of weed control.

**Table. 1. Herbicidal activity of ionic liquids with betaine and carnitine cations**

| **Compound** | ***Sinapis alba*** | ***Chenopodium album*** |
|---|---|---|
| | weed control (%) ---- | |
| [Bet][2,4-D] | 32 | 27 |
| [Bet] [MCPA] | 38 | 21 |
| [Car][2,4-D] | 32 | 7 |
| [Car][MCPA] | 38 | 34 |
| Herbicide X | 40 | 4 |
| Herbicide Y | 25 | 20 |

All tested ionic liquids were found more effective than commercial herbicides in controlling common lambsquarters, while only [Bet][2,4-D] and [Car][2,4-D] had slightly lower activity in reduction of growth of white mustard.

**Table. 2. Herbicidal activity of ionic liquids with dodecyldimethylbetaine cation**

| **Compound** | ***Centaurea cyanus*** | ***Chenopodium album*** |
|---|---|---|
| | -------- weed control (%) --------- | |
| [C₁₂Bet][2,4-D] | 54 | 65 |
| [C₁₂Bet][MCPA] | 75 | 62 |
| Herbicide X | 18 | 46 |
| Herbicide Y | 0 | 47 |

Both tested compounds [C₁₂Bet][2,4-D] and [C₁₂Bet][MCPA] reduced growth of cornflower and common lambsquarters more efficiently in comparison with commercial herbicides.

### Tests of herbicidal activity in field experiments

Trials were performed on the plots of the size 2 m x 5 m. Spray solutions of tested ionic liquids were prepared by dissolving in water:isopropanol (1:1) mixture a measured amount of the compound corresponding to 400 g of MCPA (calculated as the anion) per 1 ha. The following ionic liquids of the invention were tested:
- [Bet][MCPA] - betaine (4-chloro-2-methylphenoxy)acetate,
- [Car][MCPA] - carnitine (4-chloro-2-methylphenoxy)acetate,
- [C₁₂Bet][MCPA] - dodecyldimethylglycine (4-chloro-2-methylphenoxy)acetate,

Commercial herbicide containing MCPA (300 g of the active substance as sodium-potassium salt in 1 dm³) designated as Herbicide Y was used as the reference product. This herbicide was dissolved in water (in accordance with manufactures recommendation) in the amount corresponding to 400 g MCPA per 1 ha. Controls were plots without herbicide treatment.

Treatments were performed using backpack sprayer equipped with flat-fan nozzles Tee Jet 110 03 XR, under constant pressure of 0.2 MPa and spray volume of 200 dm³ per 1 ha. At the time of treatment, weeds were at the following growth stages: common lambsquarters (*Chenopodium album*) - from 4 to 10 leaves, field penny-cress (*Thlaspi arvense*) - blossom phase.

Typical methodology adopted for this type of test was used to determine efficacy of tested compounds, i.e. degree of eradication of a given weed species on each plot treated with tested compound in comparison with corresponding untreated plot was assessed. Efficacy of weeds control were presented as percent data, where 100% means total weeds destruction (weeds completely dried), and 0% means lack of herbicidal activity. The results were presented as a mean of assessment of weeds control from four replicates.

**Table. 3. Herbicidal activity of ionic liquids with betaine and carnitine cations in common lambsquarters and field penny-cress.**

| **Compound** | ***Thlaspi arvense*** | ***Chenopodium album*** |
|---|---|---|
| | --------------- weed control (%) ---------------- | |
| [Bet][MCPA] | 35 | 81 |
| [Car][MCPA] | 38 | 82 |
| Herbicide Y | 18 | 27 |

**Table. 4. Herbicidal activity of ionic liquids with betaine, carnitine and dodecyldimethylbetaine cations in common lambsquarters**

| **Compound** | ***Chenopodium album*** |
|---|---|
| | --- weed control (%) --- |
| [Bet][MCPA] | 100 |
| [Car][MCPA] | 100 |
| [C₁₂Bet][MCPA] | 100 |
| Herbicide Y | 90 |

Field experiments showed that ionic liquids containing MCPA anion (Tables 3 and 4) were more biologically effective comparing to presently used commercial herbicide formulation containing MCPA as sodium-potassium salts. Herbicidal ionic liquids did not cause damage of spring wheat and spring barley plants.

## Claims

1. A ionic liquid compound of the general formula (I) 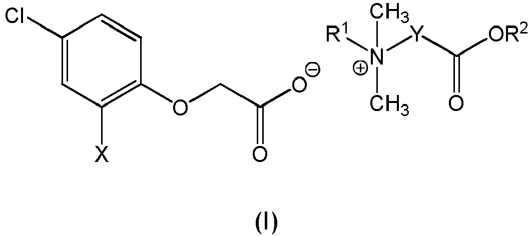 wherein
- X represents Cl or CH₃,
- Y represents -CH₂- or -CH₂-CH(OH)-CH₂-,
- R¹ represents straight chain alkyl C1 to C18 or their mixtures, cocoamidopropyl, or straight chain C1-C18-alkylamidopropyl or their mixtures, and
- R² represents hydrogen atom or straight chain alkyl C1 to C18 or their mixtures.

2. The compound according to claim 1, wherein X represents Cl.

3. The compound according to claim 1, wherein X represents CH₃.

4. The compound according to any one of claims 1 to 3, wherein Y represents - CH₂-.

5. The compound according to any one of claims 1 to 3, wherein Y represents -CH₂-CH(OH)-CH₂-.

6. The compound according to any one of claims 1 to 5, wherein R¹ represents C1 alkyl.

7. The compound according to any one of claims 1 to 5, wherein R¹ represents C12 or C14 alkyl.

8. The compound according to any one of claims 1 to 5, wherein R¹ represents a mixture of C12 and C14 alkyls.

9. The compound according to any one of claims 1 to 5, wherein R¹ represents cocoamidopropyl.

10. The compound according to any one of claims 1 to 9, wherein R² represents hydrogen atom.

11. The compound according to any one of claims 1 to 9, wherein X represents Cl, R² represents straight chain alkyl C1 to C18, especially straight chain C12 alkyl.

12. The compound according to claim 1, wherein X represents CH₃, Y represents -CH₂-, R¹ represents C1 alkyl, and R² represents H.

13. A herbicidal composition, comprising as herbicidally active ingredient a compound of formula (I) as defined in any one of claims 1 to 12, optionally in combination with a second another herbicidally active ingredient.

14. The composition according to claim 14, wherein the second another herbicidally active ingredient is selected from sodium salt, potassium salt, ammonium salt or an ester of a herbicidal compound selected from carboxylic acids, phosphonic acids and their mixtures.

15. The composition according to claim 13 or 14, which further comprises a wetting agent, a thickener, an antifoaming agent, a homogenizer or their mixtures.

## Patentansprüche

1. Eine ionische flüssige Verbindung der allgemeinen Formel (I) 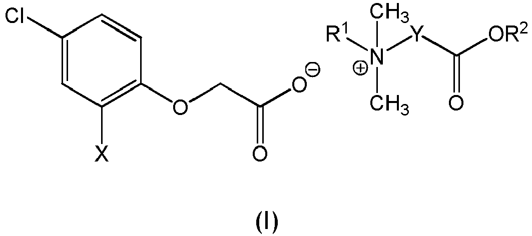 worin
- X für Cl oder CH₃ steht,
- Y für -CH₂- oder -CH₂-CH(OH)-CH₂- steht,
- R¹ für geradkettiges Alkyl C1 bis C18 oder deren Mischungen, Cocoamidopropyl, oder (geradkettiges C1-C18 Alkyl)amidopropyl oder deren Mischungen steht,
- R² für ein Wasserstoffatom oder geradkettiges Alkyl C1 bis C18 oder deren Mischungen steht.

2. Verbindung nach Anspruch 1, wobei X für Cl steht.

3. Verbindung nach Anspruch 1, wobei X für CH₃ steht.

4. Verbindung nach einem der Ansprüche 1 bis 3, wobei Y für -CH₂- steht.

5. Verbindung nach einem der Ansprüche 1 bis 3, wobei Y für -CH₂-CH(OH)-CH₂- steht.

6. Verbindung nach einem der Ansprüche 1 bis 5, wobei R¹ für C1 Alkyl steht.

7. Verbindung nach einem der Ansprüche 1 bis 5, wobei R¹ für C12 oder C14 Alkyl steht.

8. Verbindung nach einem der Ansprüche 1 bis 5, wobei R¹ für eine Mischung von C12- und C14-Alkylgruppen steht.

9. Verbindung nach einem der Ansprüche 1 bis 5, wobei R¹ für Cocoamidopropyl steht.

10. Verbindung nach einem der Ansprüche 1 bis 9, wobei R² für ein Wasserstoffatom steht.

11. Verbindung nach einem der Ansprüche 1 bis 9, wobei X für Cl, R² für geradkettiges Alkyl C1 bis C18, insbesondere geradkettiges C12-Alkyl, stehen.

12. Verbindung nach Anspruch 1, wobei X für CH₃, Y für -CH₂-, R¹ für C1 Alkyl, und R² für ein Wasserstoffatom, stehen.

13. Herbizide Zusammensetzung, umfassend als herbizide Wirkstoff eine Verbindung der Formel (I), wie in einem der Ansprüche 1 bis 12 definiert, gegebenenfalls in Kombination mit einem zweiten anderen herbiziden Wirkstoff.

14. Zusammensetzung nach Anspruch 14, wobei der zweite andere herbizide Wirkstoff ausgewählt ist aus Natriumsalz, Kaliumsalz, Ammoniumsalz oder einem Ester einer herbiziden Verbindung, ausgewählt aus Carbonsäuren, Phosphonsäuren und deren Mischungen.

15. Zusammensetzung nach Anspruch 13 oder 14, die weiterhin ein Benetzungsmittel, ein Verdickungsmittel, ein Antischaummittel, einen Homogenisator oder deren Gemische enthält.

## Revendications

1. Composé liquide ionique de formule générale (I) 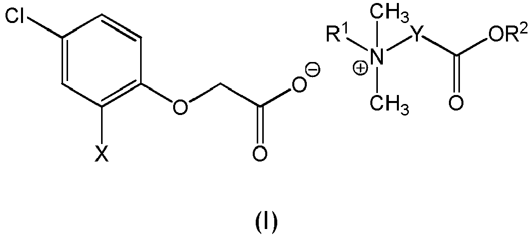 dans lequel
- X représente Cl ou CH₃,
- Y représente -CH₂- ou -CH₂-CH(OH)-CH₂-,
- R¹ représente un C1 à C18 alkyle à chaîne droite ou leurs mélanges, cocoamidopropyle, ou (C1-C18 alkyle)amidopropyle à chaîne droite, ou leurs mélanges,
- R² représente un atome d'hydrogène ou un alkyle C1 à C18 à chaîne droite, ou leurs mélanges.

2. Composé selon la revendication 1, dans lequel X représente Cl.

3. Composé selon la revendication 1, dans lequel X représente CH₃.

4. Composé selon l'une quelconque des revendications 1 à 3, dans lequel Y représente -CH₂-.

5. Composé selon l'une quelconque des revendications 1 à 3, dans lequel Y représente -CH₂-CH(OH)-CH₂-.

6. Composé selon l'une quelconque des revendications 1 à 5, dans lequel R¹ représente un alkyle en C1.

7. Composé selon l'une quelconque des revendications 1 à 5, dans lequel R¹ représente un alkyle en C12 ou C14.

8. Composé selon l'une quelconque des revendications 1 à 5, dans lequel R¹ représente un mélange d'alkyles en C12 et C14.

9. Composé selon l'une quelconque des revendications 1 à 5, dans lequel R¹ représente un cocoamidopropyle.

10. Composé selon l'une quelconque des revendications 1 à 9, dans lequel R² représente un atome d'hydrogène.

11. Composé selon l'une quelconque des revendications 1 à 9, dans lequel X représente Cl, R² représente un C1 à C18alkyle à chaîne droite, en particulier un alkyle en C12 à chaîne droite.

12. Composé selon la revendication 1, dans lequel X représente CH₃, Y représente -CH₂-, R¹ représente un alkyle en C1, et R² représente un atome d'hydrogène.

13. Composition herbicide, comprenant en tant qu'ingrédient herbicide actif un composé de formule (I) tel que défini dans l'une quelconque des revendications 1 à 12, facultativement en combinaison avec un second autre ingrédient herbicide actif.

14. Composition selon la revendication 14, dans laquelle le second autre ingrédient herbicide actif est choisi parmi le sel de sodium, le sel de potassium, le sel d'ammonium ou un ester d'un composé herbicide choisi parmi les acides carboxyliques, les acides phosphoniques et leurs mélanges.

15. Composition selon la revendication 13 ou 14, qui comprend en outre un agent mouillant, un épaississant, un agent antimousse, un homogénéisateur ou leurs mélanges.
